## Europäisches Patentamt
## European Patent Office
## Office européen des brevets

(19)

(11) Veröffentlichungsnummer: **0 011 878**
**B1**

(12) # EUROPÄISCHE PATENTSCHRIFT

(45) Veröffentlichungstag der Patentschrift:
22.12.82

(21) Anmeldenummer: **79104852.3**

(22) Anmeldetag: **03.12.79**

(51) Int. Cl.³: **G 01 P 5/00, G 01 F 1/66, G 01 S 15/58, A 61 B 10/00**

(54) **Verfahren und Vorrichtung zur Bestimmung von Strömungsgeschwindigkeiten sowie deren Verwendung in einem Ultraschall-Diagnostikgerät.**

(30) Priorität: **04.12.78 CH 12361/78**

(43) Veröffentlichungstag der Anmeldung:
**11.06.80 Patentblatt 80/12**

(45) Bekanntmachung des Hinweises auf die Patenterteilung:
**22.12.82 Patentblatt 82/51**

(84) Benannte Vertragsstaaten:
**CH DE FR GB IT NL SE**

(56) Entgegenhaltungen:
**FR-A-2 375 764**
**US-A-3 914 999**

**BIOMEDIZINISCHE TECHNIK, Band 21, (Supplement), Juni 1976, Berlin, DE, R. FEHR et al.: "Pulsed ultrasonic doppler veloclmeter for measuring velocity profiles by analog signal processing methods", Seiten 289, 290**

**ELECTRONICS LETTERS, Band 11, Nr. 8, 17. April 1975, Hitchin, Herts R.C. HANSEN et al.: "Ultrasonic blood flowmeter yielding instantaneous velocity profile by realtime phase detection", Seiten 183, 184**

**BIOMEDIZINISCHE TECHNIK, Band 21, (Supplement), Juni 1976, Berlin, DE, M. BRANDESTINE: "A digital 128-channel transcutaneous bloodflowmeter", Seiten 291, 292**

(73) Patentinhaber: **F. HOFFMANN-LA ROCHE & CO. Aktiengesellschaft, CH-4002 Basel (CH)**

(72) Erfinder: **Fehr, Rainer, Ringstrasse 2, CH-4153 Reinach (CH)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

# Verfahren und Vorrichtung zur Bestimmung von Strömungsgeschwindigkeiten sowie deren Verwendung in einem Ultraschall-Diagnostikgerät

Die Erfindung betrifft ein Verfahren der im Oberbegriff des Anspruchs 1 angegebenen Art. Ein solches Verfahren ist aus DE-A 2 703 879 bekannt.

Bei einem anderen bekannten Verfahren der oben erwähnten Art (Amerikanische Patentschrift 3 914 999) wird der Momentanwert der Projektion der Punktgeschwindigkeit in einer vorbestimmten Richtung X durch die Beziehung $V_x = k\Delta\varphi/\Delta t$ bestimmt, worin $k$ = Proportionalitätskonstante, $\Delta\varphi$ = Phasendifferenz zwischen den Dopplersignalen, und $\Delta t$ = ein ganzzahlig Vielfaches der Pulswiederholungsfrequenz ist.

Zur Verbesserung des Signal/Rauschen-Verhältnisses werden die Messergebnisse über eine gewisse Zeit ausgemittelt. Dann gilt

$$\bar{V}_x \, \alpha \, \frac{1}{n} \cdot \sum_{k=1}^{n} \frac{\Delta\varphi_k}{\Delta t} \text{ wobei } \Delta\varphi_k = \varphi_{k+1} - \varphi_k$$

$\varphi_k$ ist die Phase des Dopplersignals nach dem k-ten Sendepuls, wenn $\Delta t = \frac{1}{PRF}$ mit PRF = Impulswiederholfrequenz (pulse repetition frequency).

Bei der transcutanen Messung des Geschwindigkeitsprofils einer Blutströmung mit dem soeben erwähnten Verfahren, stellt sich das Problem, den Mittelwert der Phasendifferenz $\overline{\Delta\varphi}$ zwischen den Dopplersignalen, bei einem schlechten Signal/Rauschen-Verhältnis, mit grösstmöglicher Genauigkeit und in einem vorbestimmten Zeitraum zu bestimmen. (Dass das Signal/Rauschen-Verhältnis dabei schlecht ist, ist leicht verständlich, weil die Echos von den Blutpartikeln naturgemäss wesentlich schwächer als diejenigen von den stationären umgebenden Strukturen sind). Zur Lösung dieses Problems wurde bereits vorgeschlagen (M. Brandestini, "Topoflow-A Digital Full Range Doppler Velocity Meter", IEEE Transactions on Sonics and Ultrasonics, September 1978, VI. SU-25, Nr. 5, pp. 287–293), einen Discriminator zu verwenden, der aus einem "zero crossing detector" und einem diesem nachgeschalteten sweep integrator besteht.

Dieser bekannte Diskriminator weist folgende Nachteile auf:

1. Der gewünschte lineare Zusammenhang zwischen der Phasendifferenz und dem Ausgangswert des Diskriminators besteht nicht über den theoretisch möglichen Bereich von $2\pi$.

2. Ein abnehmendes Signal/Rausch-Verhältnis führt zu einer systematischen Verkrümmung der Kennlinie, was in der Praxis unkontrollierbare Messfehler verursacht.

3. Es werden nur skalare Werte der Phasendifferenz ermittelt, so dass der Wert $\Delta\varphi = \pi$ im Vorzeichen unbestimmt ist.

Der Erfindung liegt die Aufgabe zugrunde, diese Nachteile zu beseitigen.

Erfindungsgemäss wird dies durch die im Kennzeichen des Anspruchs 1 genannten Merkmale erreicht.

Gegenstand der Erfindung ist ferner eine Vorrichtung zur Durchführung des erfindungsgemässen Verfahrens, wie sie in Anspruch 2 angegeben ist.

Ein weiterer Gegenstand der Erfindung ist die Verwendung des erfindungsgemässen Verfahrens in einem Ultraschall-Diagnostikgerät, das zur Bestimmung eines Geschwindigkeitsprofils einer Strömung dient, insbesondere der Blutströmung in einem Blutgefäss.

Die wesentlichen Vorteile, die mit dem erfindungsgemässen Verfahren erzielt werden, liegen darin, dass das Messergebnis weitgehend frei von systematischen Fehlereinflüssen ist und dass der theoretisch maximale Messbereich ganz ausgenutzt wird.

Im folgenden werden anhand der beiliegenden Zeichnungen einige Ausführungsbeispiele der Erfindung beschrieben. Es zeigen

Fig. 1 das Blockschaltbild eines Ultraschall-Doppler-Geräts zur Bestimmung des Geschwindigkeitsprofils einer Strömung,

Fig. 2–6 typische Signalverläufe an mehreren Stellen der Fig. 1,

Fig. 7 das Blockschaltbild des Festzeichenlöschers 43 und der Signalaufbereitungseinheit 44 des Geräts nach Fig. 1,

Fig. 8–11 eine schematische Darstellung der Signalverläufe an mehreren Stellen der Fig. 7,

Fig. 12 die prinzipielle Struktur des Diskriminators 73 in der Fig. 7,

Fig. 13 vier mögliche Phasendifferenzvektore,

Fig. 14 das Blockschaltbild eines Schieberegisters,

Fig. 15 die Struktur des Schieberegisters in Fig. 14,

Fig. 16 Schaltungen, die den Eingängen des Diskriminators 73 unmittelbar vor- bzw. nachgeschaltet sind,

Fig. 17 die Wahrheitstabelle für die Verknüpfung von binärcodierten Eingangssignalen im Phasendifferenzdetektor 11,

Fig. 18, 19 die Realisierung der Verknüpfung gemäss der Wahrheitstabelle in Fig. 17 mit Gattern,

Fig. 20, 21 die Realisierung der Verknüpfung gemäss der Wahrheitstabelle in Fig. 17 mit einem PROM,

Fig. 22 das Blockschaltbild des Tiefpassfilters 12 (oder 13) in Fig. 12,

Fig. 23 die Realisierung der Recheneinheit 14 in Fig. 12 mit einem ROM,

Fig. 24 die Realisierung der Verknüpfung gemäss der Wahrheitstabelle in Fig. 25 mit einem PROM,

Fig. 25 die Wahrheitstabelle für die Verknüpfung von ternärcodierten Eingangssignalen im Phasendifferenzdetektor 11,

Fig. 26 eine Anordnung zur Messung der Amplitude-Frequenz-Charakteristiken eines Dopplerfrequenzdetektors,

Fig. 27 gemessene Geschwindigkeit-Frequenz-Charakteristiken eines "zero crossing counters",

Fig. 28 gemessene Geschwindigkeit-Frequenz-Charakteristik der Anordnung nach Fig. 7,

Fig. 29 eine Ausgestaltung der Recheneinheit 14 zur Erzeugung einer Turbulenzanzeige,

Fig. 30 eine schematische Darstellung einer mit dem erfindungsgemässen Gerät erzeugten Anzeige,

Fig. 31 die Realisierung des Phasendifferenzdetektors 11 in Fig. 12 mit einem PROM oder mit einer PROM-Anordnung,

Fig. 32 eine vereinfachte Anordnung zur Realisierung des Phasendifferenzdetektors 11 mit PROM-Einheiten.

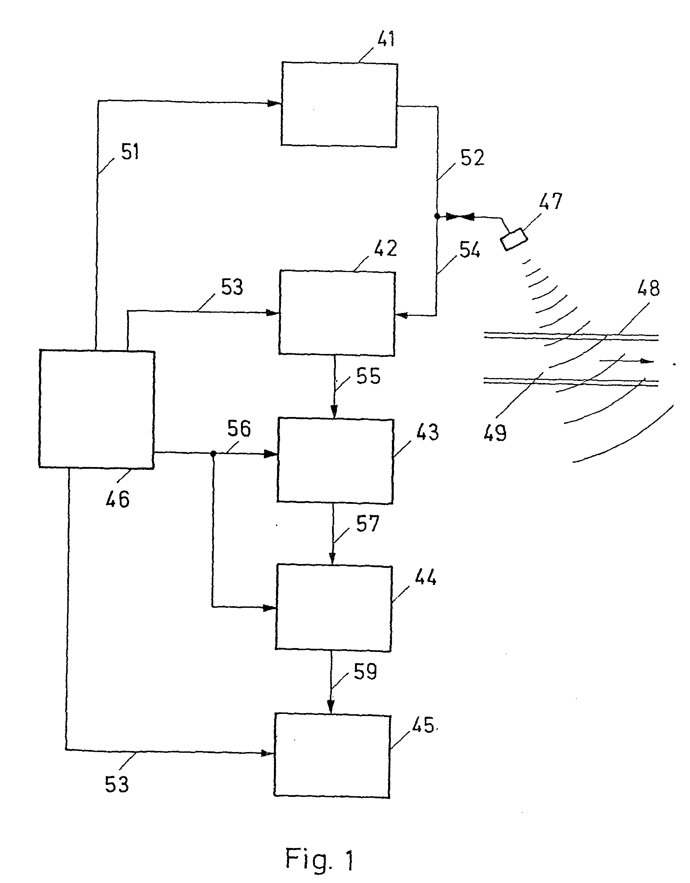

Fig. 1 zeigt das Blockschaltbild eines Ultraschall-Doppler-Geräts zur Bestimmung des Geschwindigkeitsprofils eines fliessenden Fluids (wie einer Flüssigkeit, z.B. Blut, oder eines Gases, z.B. von Luft). Das Prinzip dieses Geräts ist in der deutschen Offenlegungsschrift DE-A-2 406 630 und in der entsprechenden amerikanischen Patentschrift US-A-3 914 999 beschrieben. Nach diesem bekannten Prinzip wird ein z.B. durch ein Rohr 48 fliessendes Fluid 49 mit zwei aufeinanderfolgenden, von einem Wandler 47 abgegebenen Ultraschallimpulse bestrahlt, die entsprechenden Doppelfrequenz verschobenen Echos von Reflektoren im Fluid entlang des Ultraschallstrahls werden mit demselben Wandler 47 empfangen, und aus den Phasenunterschieden zwischen Echos gleicher Laufzeit vom ersten und zweiten gesendeten Ultraschallimpuls wird durch geeignete Signalverarbeitung ein Signal 59 abgeleitet, dessen zeitlicher Verlauf dem Geschwindigkeitsprofil des Fluids im untersuchten Querschnitt entspricht. Die soeben erwähnte Signalverarbeitung wird in der Anordnung nach Fig. 1 durch die Reihenschaltung eines Empfängers 42, eines Festzeichenlöschers 43 und einer Signalaufbereitungseinheit 44 durchgeführt, die das dem Geschwindigkeitsprofil entsprechende Signal 59 einer geeigneten Anzeigeeinheit 45, z.B. einem Kathodenstrahloszillographen zuführt. Eine zentrale Steuereinheit 46 steuert die Funktion der Einheiten der Anordnung nach Fig. 1.

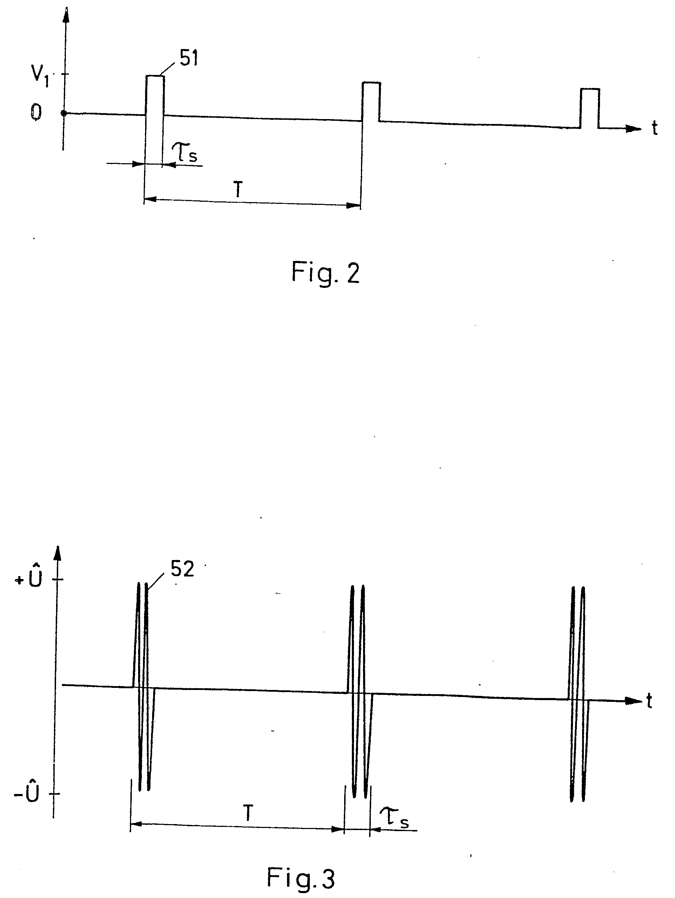

Im Betrieb wird der Sender 41 durch Steuerimpulse 51 von der Steuereinheit zur Abgabe von Sendeimpulsen 52 an den Wandler 47 angeregt. Die Steuerimpulse 51 (siehe Fig. 2) haben beispielsweise eine Dauer $\gamma_s$ = 0,5 µs und eine Pulswiederholungsperiode T = 100 µs. Die entsprechenden Senderimpulse 52 (siehe Fig. 3) sind Wellenimpulse mit einer Senderspannung Û = 20V und einer Sendefrequenz fo = 4 MHz, so dass in diesem Beispiel die Anzahl gesendeter Schwingungen pro Impuls fo · $\gamma_s$ = 2 ist.

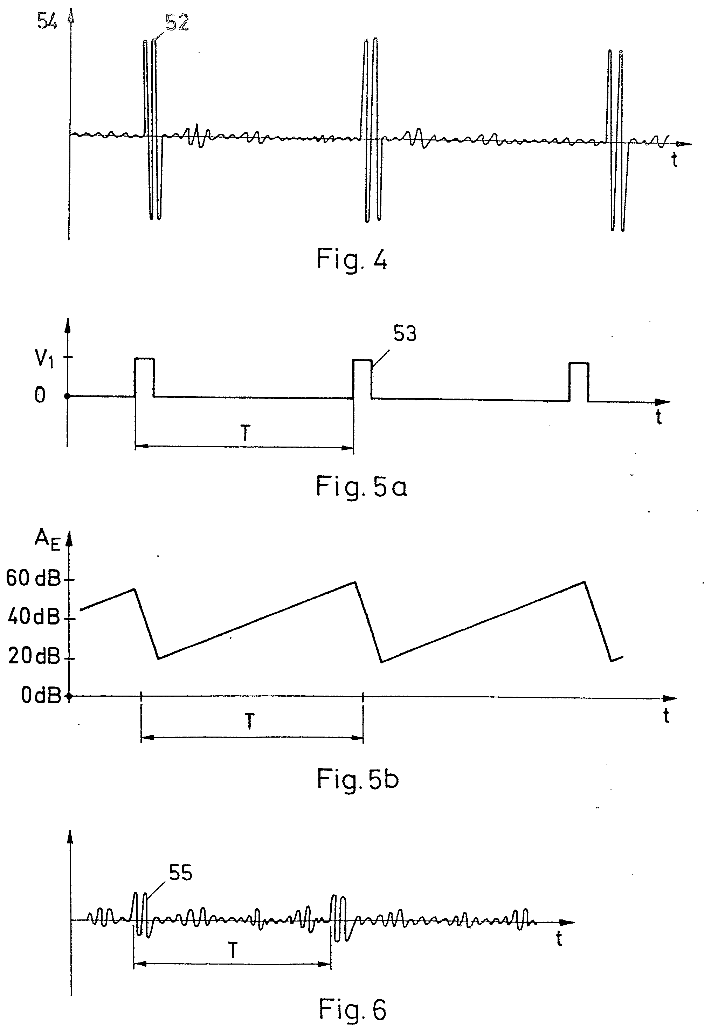

Fig. 4 zeigt ein typisches Eingangssignal 54 des Empfängers 42, welches aus Sendeimpulsen 52 und verschiedenen dopplerfrequenten Echosignalen besteht.

Da die Amplituden der vom Wandler 47 an den Empfänger 42 abgegebenen Echosignale von der Entfernung zwischen dem Wandler und dem jeweiligen Reflektor abhängen, und somit von der Laufzeit, d.h. von dem Zeitintervall, welches seit dem Aussenden eines Sendeimpulses und dem Empfangen des Echos eines Reflektors vergangen ist, wird die Verstärkung des Signals 54 im Empfänger entsprechend geregelt, um die durch die Entfernung zwischen dem Wandler und dem jeweiligen Reflektor bedingte Dämpfung aufzuheben. Wie in Fig. 5a und 5b gezeigt, wird der Verlauf der geregelten Verstärkung $A_E$ im Empfänger 42 durch Steuerimpulse 53 mit den Sendeimpulsen synchronisiert. Im Empfänger 42 wird ausserdem die Amplitude der im Empfangssignal 54 enthaltenen Sendeimpulse begrenzt. Fig. 6 zeigt eine schematische Darstellung des Verlaufs eines typischen, durch die oben erwähnten Massnahmen (geregelte Verstärkung und Begrenzung) erzeugten Ausgangssignals 55 des Empfängers.

Das Ausgangssignal 55 besteht aus doppelfrequenzverschobenen Echosignalen, die die Geschwindigkeitsinformation tragen, und aus Echosignalen, welche von relativ unbeweglichen Körperstrukturen erzeugt werden, z.B. von der Wand eines Blutgefässes, wenn ein Geschwindigkeitsprofil des Blutstroms gemessen wird. Die letztgenannten Echosignale, die sogenannten stationären Echosignale oder Festechos, sind meistens starke Störsignale, die den relativ schwachen doppelfrequenzverschobenen Echosignalen überlagert sind. Für die Erzeugung von Geschwindigkeitsprofilen mit der aus der DE-A-2 406 630 bzw. US-A-3 914 999 bekannten Verfahren ist es daher notwendig, ein periodisches Filter in der Art eines Festzeichenlöschers 43 (gebräuchliche Bezeichnung in der Radartechnik) zu benutzen, welches im Stande ist, die schwachen, dopplerfrequenzverschobenen Echosignale von den viel stärkeren, überlagerten stationären Echosignalen zu trennen. Hierfür wird vorzugsweise die in der DE-A-2 703 879 beschriebene Filteranordnung als Festzeichenlöscher verwendet. Dieser Festzeichenlöscher dämpft für jeden Punkt im Fluid entlang dem Ultraschallstrahl die stationären Echoanteile sehr stark, lässt jedoch schon Echosignale mit kleinen Dopplerfrequenzen praktisch ungedämpft durch, so dass die Messung kleiner Strömungsgeschwindigkeiten und damit die Messung des Geschwindigkeitsprofils einer Strömung mit hoher Empfindlichkeit und Genauigkeit möglich ist.

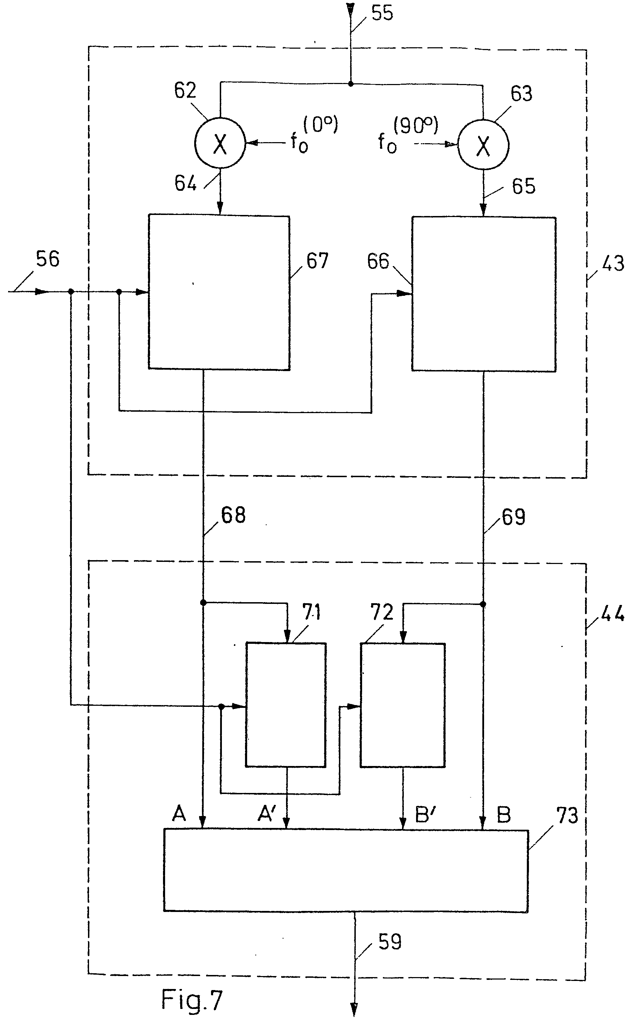

In Fig. 7 werden der Festzeichenlöscher 43 und die Signalaufbereitungseinheit 44 der Anordnung nach Fig. 1 genauer dargestellt.

Der Festzeichenlöscher 43 enthält zwei Synchron-Demodulatoren 62, 63 und zwei periodische Hochpassfilter 66, 67 gemäss der DE-A-2 703 879. Wie in Fig. 7 gezeigt, wird das Eingangssignal 55 des Festzeichenlöschers 43 mit den Demodulatoren 62, 63 in Quadratur demoduliert, d.h. das Eingangssignal 55 wird mit zwei gegeneinander um 90° phasenverschobenen Referenzsignalen fo (0°) und fo (90°) multipliziert, um zwei Niederfrequenzsignale 64, 65 zu erzeugen. Da bei der Demodulation auch höhere Frequenzen erzeugt werden, enthalten die Demodulatoren Tiefpassfilter, die nur die Niederfrequenzsignale 64, 65 durchlassen.

Diese Quadratur-Demodulation ist aus folgenden Gründen angezeigt. Sie ist für ein Dopplergerät zur Bestimmung des Geschwindigkeitsprofils einer Strömung notwendig, wenn man die Strömungsrichtung aus dem demodulierten Signal ableiten will. Die Quadratur-Demodulation ist auch vorteilhaft, wenn, wie im vorliegenden Fall, die Festzeichenlöschung unter Verwendung eines abgetasteten Filters durchgeführt wird, da man durch die Quadratur-Demodulation sowohl die Abtastfrequenz dieses Filters als auch die Anzahl der benötigten Speicherplätze so niedrig wie möglich halten kann.

Die Wirkungsweise des Festzeichenlöschers 43 ist wie folgt:

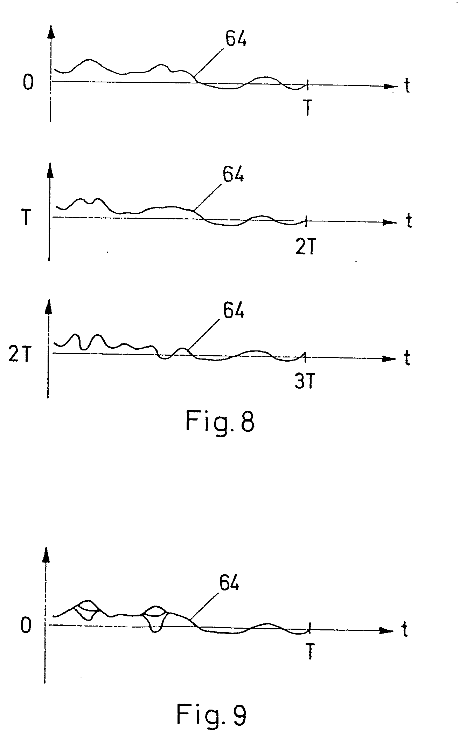

Fig. 8 zeigt eine schematische Darstellung des demodulierten Signals 64 am Eingang des Filters 67. Das demodulierte Signal 65 am Eingang des Filters 66 sieht gleich wie das Signal 64 aus, mit dem Unterschied, dass die Dopplerschwingung je nach Strömungsrichtung um $+90°$ oder $-90°$ phasenverschoben ist. In Fig. 8 sind die Echozüge von mehreren aufeinanderfolgenden Pulsen untereinander dargestellt. Fig. 9 zeigt dieselben Echos übereinander. Der Übersichtlichkeit halber werden in Fig. 9 nur zwei Stellen gezeigt, in denen eine Dopplerschwingung auftritt, während der Rest des Echosignalverlaufs stationär bleibt.

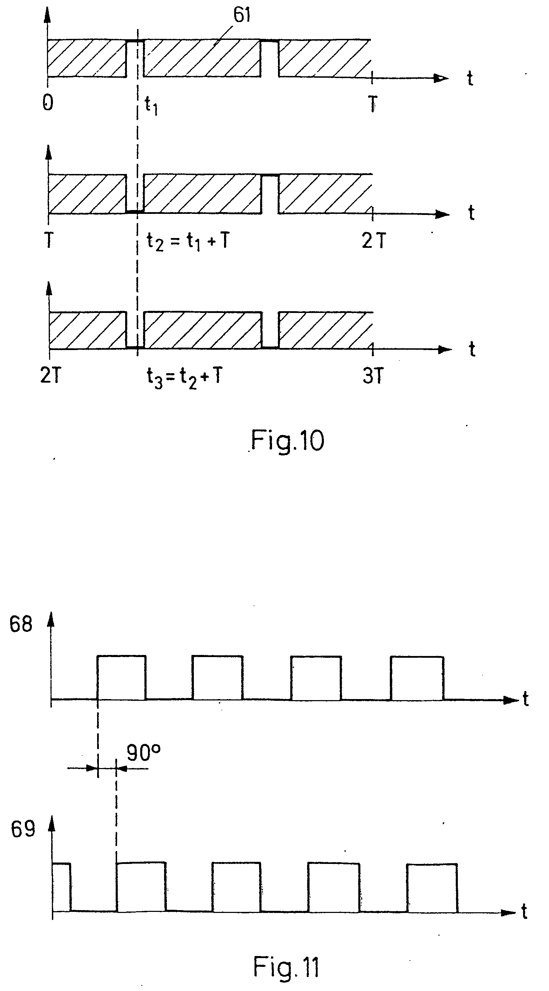

Die Signalverarbeitung in den periodischen Hochpassfiltern 66, 67 ist in der DE-A-2 703 879 ausführlich beschrieben. Die Ausgangssignale 68, 69 dieser Filter werden nun anhand der Fig. 10 und 11 erläutert.

Da die Ausgangssignale praktisch den gleichen Verlauf haben und nur in der Phase unterschiedlich sind, zeigt Fig. 10 als Beispiel das Ausgangssignal 68 vom Filter 67 in Fig. 7. Diese abgetasteten Werte entsprechen dem Vorzeichen der in Fig. 9 gezeigten Dopplerschwingungen. Wie in Fig. 10 für die erste der Dopplerschwingungen gezeigt, erfolgt die Abtastung der Werte für einen bestimmten Punkt des Geschwindigkeitsprofils mit der Periode T der gesendeten Ultraschallpulse. Jeder abgetastete Wert ist z.B. ein 1-Bit-Signal mit der Dauer eines Abtastimpulses. Durch die Wirkung des Festzeichenlöschers ist es möglich, beide Dopplerschwingungen in Fig. 9 zu detektieren. In Fig. 10 sind die schraffierten Zonen 61 unbestimmt, da, wenn keine Dopplerschwingungen vorhanden sind, das Vorzeichen des Ausgangssignals 68 nicht eindeutig definiert ist, weil die Werte der Ausgangssignale 68, 69 nahe bei Null liegen und von Störsignalen überlagert ist.

Fig. 11 zeigt einen schematischen Verlauf der Ausgangssignale 68, 69 der Filter 66, 67 (in Fig. 7) für den angenommenen Fall, dass nur für einen Punkt des Geschwindigkeitsprofils eine Dopplerfrequenz abgetastet wird. Diese Annahme ermöglicht die Ausgangssignale 68, 69 übersichtlich darzustellen. Die Richtung der Phasenverschiebung zwischen den sonst gleichverlaufenden Signalen 68, 69 entspricht dem Vorzeichen, d.h. der Richtung, der Punktgeschwindigkeit im untersuchten Punkt. Wegen der durchgeführten Quadratur-Modulation sind die Signale 68, 69 orthogonale Vektorkomponenten des Dopplerschwingungsvektors.

Wie in der Fig. 7 gezeigt, werden die Ausgangssignale 68, 69 des Festzeichenlöschers 43 in der Einheit 44 verarbeitet. In dieser Einheit werden die Ausgangssignale 68, 69 mit Verzögerungsmitteln, z.B. Schieberegistern 71, 72, um eine Pulswiederholungsperiode verzögert und in einem Diskriminator 73 mit unverzögerten Ausgangssignalen 68, 69 nach dem nachstehend beschriebenen Verfahren verarbeitet, um ein analoges Ausgangssignal 59 zu erzeugen, das dem Mittelwert $\overline{\Delta\varphi}$ der Phasendifferenz zwischen Eingangssignalen 55 entspricht, die Echos gleicher Laufzeit von zwei verschiedenen Sendeimpulsen entsprechen. Der Verlauf des so erzeugten Ausgangssignals 59 entspricht dem Geschwindigkeitsprofil der Strömung in der mit Ultraschallimpulsen abgetasteten Ebene.

Am Eingang des Diskriminators 73 in der Fig. 7 werden die nicht verzögerten Signale (68, 69) A und B, und die verzögerten Signale A' und B' genannt.

Wie in Fig. 1 gezeigt, wird die Horizontalablenkung der Anzeigevorrichtung 45 durch die Steuerimpulse 53 (die gleichen wie für die Steuerung des Empfängers 42) mit der Pulswiederholungsfrequenz synchronisiert.

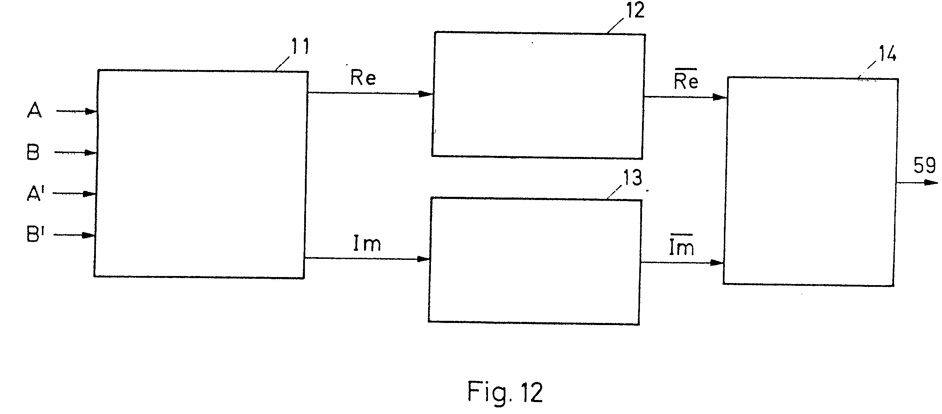

Fig. 12 zeigt die prinzipielle Struktur des Diskriminators 73 in der Fig. 7. In diesem Diskriminator werden die Eingangssignale A, B bzw. A', B' in drei Schritten verarbeitet.

Wegen der im Festzeichenlöscher 43 (siehe Fig. 7) durchgeführten Quadratur-Modulation sind die Signale A, B bzw. A', B' orthogonale Vektorkomponenten entsprechender Dopplerschwingungsvektoren, d.h. Vektoren die je eine Dopplerschwingung darstellen. A und B, bzw. A' und B', definieren also je einen Signalvektor.

Mit einem Phasendifferenzdetektor 11 werden zwei Ausgangssignale Re und Im erzeugt, die einen Phasendifferenzvektor $\overrightarrow{\Delta\varphi}$ definieren, dessen Betrag gleich 1 und dessen Phase gleich der Phasendifferenz zwischen den Signalvektoren ist, die durch A, B und A', B' definiert sind.

Allgemein formuliert und mathematisch ausgedrückt dient der Phasendifferenzdetektor dazu, mit den Eingangssignalen A, B bzw. A', B' je eine komplexe Zahl

$$Z_1 = A + j\,B$$
$$Z_2 = A' - j\,B'$$

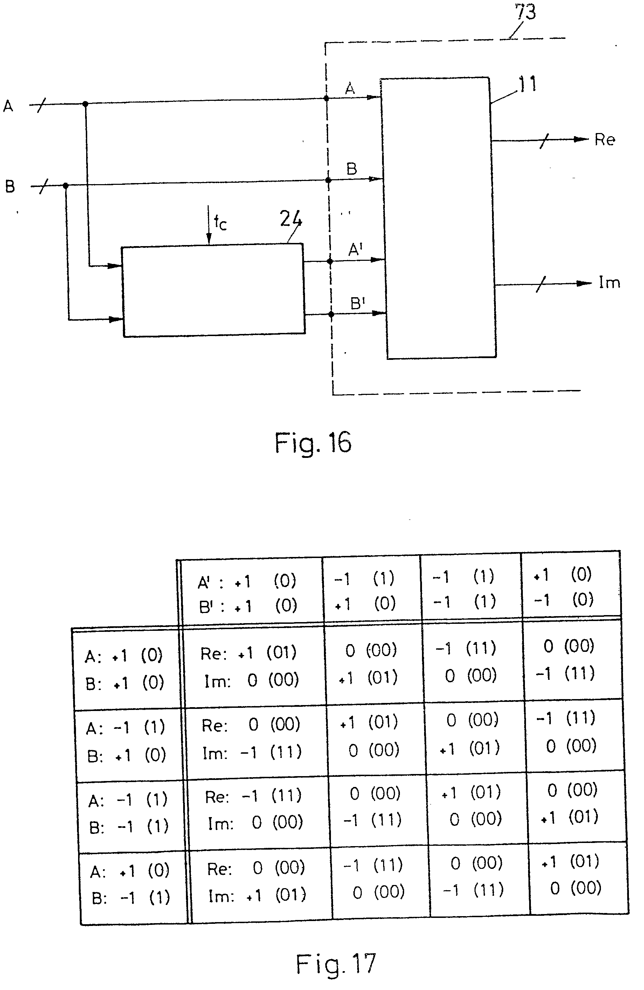

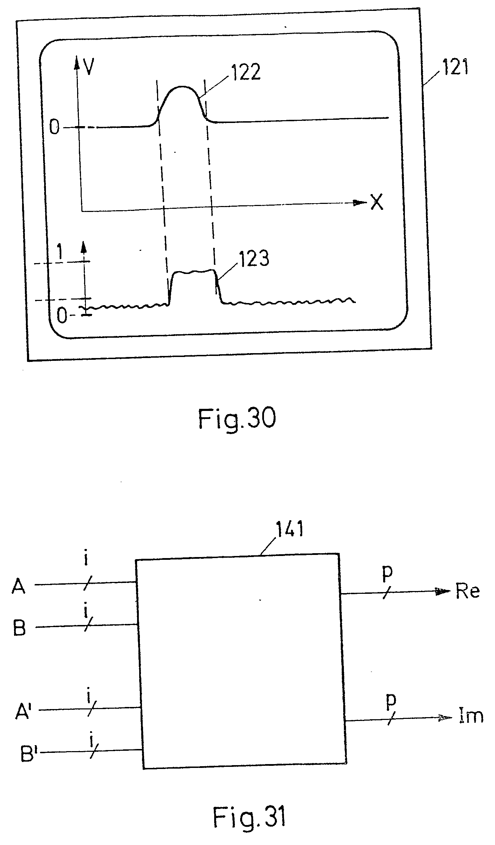

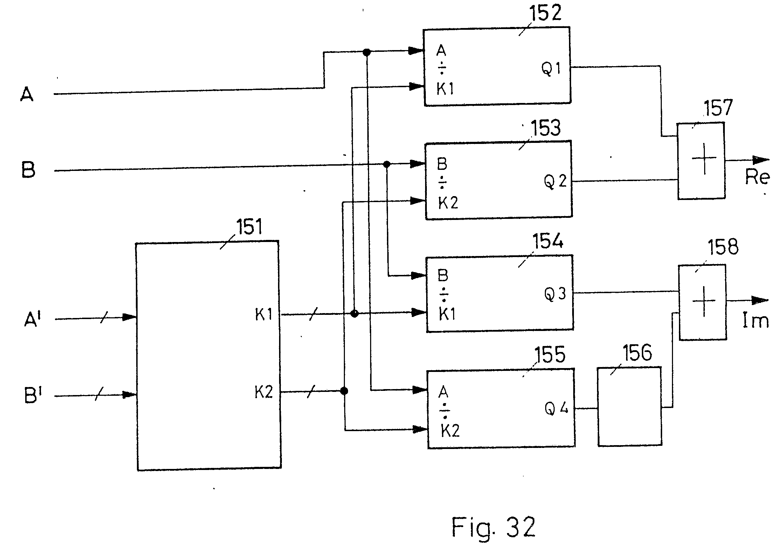

zu bilden, durch Bildung des Quotienten $Z_1/Z_2$ dieser beiden Zahlen eine komplexe Zahl zu bilden, deren Komponenten Re und Im mit den Komponenten des Phasendifferenzvektors $\overrightarrow{\Delta\varphi}$ identisch sind, und Ausgangssignale zu erzeugen, die den Komponenten Re bzw. Im entsprechen. Ein derartiger Phasendifferenzdetektor lässt sich mit verschiedenen Schaltungen realisieren. Möglichst einfache Ausführungsformen sind nachstehend anhand der Fig. 17–21 beschrieben. Allgemeine Ausführungsformen sind nachstehend anhand der Fig. 31 und 32 beschrieben.

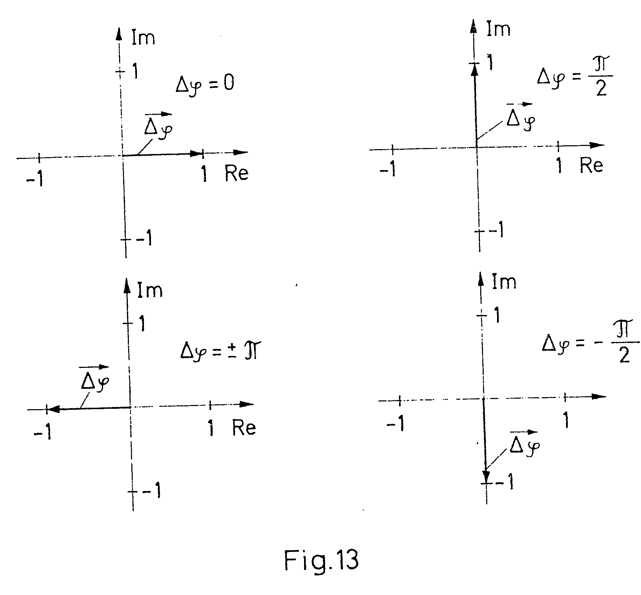

Wenn man mit begrenzten und abgetasteten Signalvektoren (A, B und A', B') arbeitet gibt es vier mögliche Phasendifferenzvektoren. Diese sind in Fig. 13 dargestellt, wobei jeder dieser vier Vektoren durch zwei Komponente Re ($\overrightarrow{\Delta\varphi}$) und Im ($\overrightarrow{\Delta\varphi}$) definiert ist. Im Gegensatz zum bisher bekannten Verfahren (M. Brandestini, «Topoflow – A Digital Full Range Doppler Velocity Meter», IEEE Transactions Sonics and Ultrasonics, September 1978, Vol. SU-25, No. 5, pp. 287–293), bei dem nur ein skalarer Wert der Phasendifferenz ermittelt wird, so dass der Wert $\Delta\varphi = \pi$ wegen dem unbestimmten Vorzeichen wenig nützlich ist, sind beim hier beschriebenen Verfahren alle Phasendifferenzvektoren eindeutig definiert.

Im zweiten Teil der Anordnung nach Fig. 12 werden die Ausgangssignale Re und Im des Phasendifferenzdetektors mit geeigneten Mitteln, z.B. mit zwei identischen, periodischen Tiefpassfiltern 12 und 13 (sogenannten «sweep integrators») ausgemittelt. Die Ausgangssignale $\overline{\text{Re}}$ bzw. $\overline{\text{Im}}$ dieser Filter entsprechen den mittleren Werten von Re bzw. Im. $\overline{\text{Re}}$ und $\overline{\text{Im}}$ definieren den mittleren Phasendifferenzvektor.

Mit der Recheneinheit 14, die den dritten Teil der Anordnung nach Fig. 12 bildet, wird das Ausgangssignal 59 des Diskriminators erzeugt. In der Recheneinheit 14 wird für jeden Punkt des zu messenden Geschwindigkeitsprofils ein der Phase des mittleren Phasendifferenzvektors entsprechendes Signal erzeugt, und von diesem Signal wird das Ausgangssignal 59 abgeleitet, dessen Verlauf dem Geschwindigkeitsprofil, z.B. der räumlichen Verteilung der gemessenen Punktgeschwindigkeiten entspricht.

Entsprechend der Ausmittelungszeit ist die Phase des mittleren Phasendifferenzvektors feiner definiert als es am Ausgang des Phasendifferenzdetektors der Fall ist, so dass um den Wert $\pi$ herum nur noch ein entsprechend kleinerer Bereich übrigbleibt, in welchem das Vorzeichen unsicher ist.

Eine Verschlechterung des Signal/Rauschen-Verhältnisses des Eingangssignals verschlechtert die Genauigkeit der Messung. Dies äussert sich in einer Reduktion des Betrages des ausgemittelten «Phasendifferenzvektors» und in einer statistischen Schwankung seiner Phase. Es tritt jedoch kein systematischer Phasenfehler auf, weil die Phase bei einem Rauschen rein zufällig ist und keinen bevorzugten Wert besitzt, gegen den sie konvergieren könnte.

Der wesentliche Unterschied zwischen dem bisher bekannten und dem neuen hier beschriebenen Verfahren lässt sich wie folgt zusammenfassen:

Da jede Punktgeschwindigkeit der Doppelfrequenz $f_D$ der von einem Punkt kommenden Echowerte entspricht, ist es auch so, dass mit dem in der Einleitung dieser Beschreibung erwähnten Verfahren ein Signal erzeugt wird, das in einem bestimmten Bereich der Dopplerfrequenz der Echosignale entspricht.

Das wesentliche Problem bei der Bestimmung der Dopplerfrequenz mittels des bekannten Verfahrens liegt in der Zuordnung der Frequenz, welche eine monotone Grösse ist, zur Phasendifferenz, welche eine periodische Grösse ist.

Bei dem bekannten Verfahren wird für jede Phasendifferenz ($\Delta\varphi_k$) die entsprechende Dopplerfrequenz $f_D = \dfrac{\Delta\varphi k}{\Delta t}$ abgeleitet und der Mittelwert einer Anzahl (z.B. 100) abgeleiteter Werte der Dopplerfrequenz berechnet. Dabei bereitet die Berechnung von $f_D = \dfrac{\Delta\varphi k}{\Delta t}$ Schwierigkeiten, wenn $\Delta\varphi$ in die Grössenordnung von $\pi$ gelangt. Besitzt $\Delta\varphi$ infolge Rauschen eine grosse statistische Schwankungsbreite, so kann um $\Delta\varphi = \pi$ herum ein Bereich entsprechend der maximalen Schwankungsbreite nicht mehr eindeutig der Frequenz zugeordnet werden. Dies führt zu einer Rausch-abhängigen Einschränkung des erlaubten $\Delta\varphi$ und schränkt somit den Messbereich beträchtlich ein.

Bei dem neuen, hier beschriebenen Verfahren werden die Komponenten des Phasendifferenzvektors (und somit der Phasendifferenzvektor) ausgemittelt, bevor die Dopplerfrequenz (die der Punktgeschwindigkeit proportional ist) davon abgeleitet wird. Dadurch wird die Schwankungsbreite von $\Delta\varphi$ beträchtlich reduziert und die Einschränkung des Messbereiches kann in der Praxis beinahe vollständig vermieden werden.

Ausführungsbeispiele des Diskriminators 73

Im folgenden werden zwei Ausführungsbeispiele des Diskriminators 73 beschrieben, die mit binärer (zweiwertiger) bzw. ternärer (dreiwertiger) Begrenzung der Eingangssignale A, B und A', B' arbeiten.

Wie aus Fig. 1 und 7 ersichtlich, ist ein Ultraschall-Pulsechogerät die Quelle für die Signale A und B. Dieses sendet Ultraschall-Impulse aus mit der Pulswiederholungsfrequenz $P_{RF}$ (typ. 10 kHz). Die Zeit zwischen den Sendepulsen wird durch die Taktfrequenz $f_c$ in N-gleiche Zeitintervalle aufgeteilt. $f_c$ ist die Abtastfrequenz und wird entsprechend der gewünschten räumlichen Auflösung gewählt (typisch $f_c = 1,28$ MHz). N ist die Anzahl Speicherplätze, welche in allen digitalen Verzögerungsleitungen gebraucht wird, die das Signal um eine volle Pulswiederholungsperiode verzögern soll (typisch N = 128).

Die nachstehend erwähnten Signalverzögerungen können durch Schieberegister oder mittels digitaler Speicher (Halbleiterspeicher, Kernspeicher) realisiert werden.

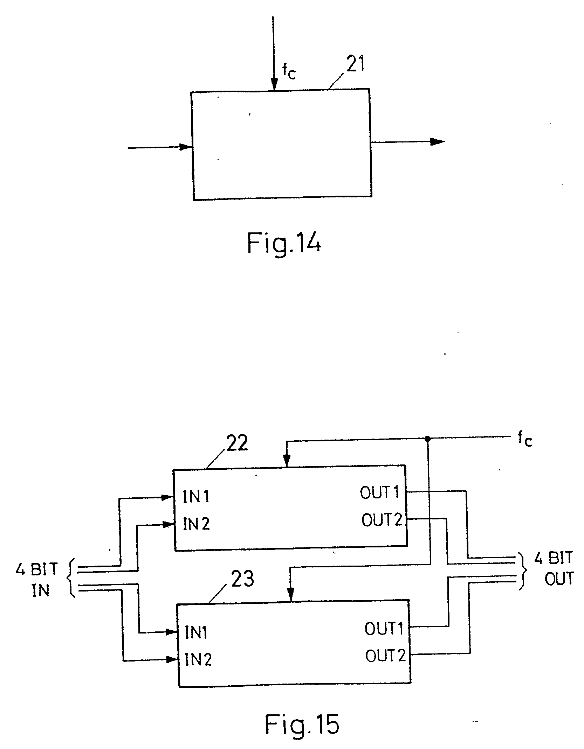

Fig. 14 zeigt das Blockschaltbild eines Schieberegisters 21 für 4-Bit-Ein- bzw. Ausgangssignale und eine Speicherkapazität von 4 × N Bit. Fig. 15 zeigt ein Beispiel der Struktur des Schieberegisters 21, wenn dieses mit der integrierten Schaltung TMS 3114 von Texas Instruments hergestellt wird, die zwei Schieberegister 22, 23 mit je 128 Speicherplätzen enthält. Schieberegister mit einer derartigen Struktur werden in den nachstehend beschriebenen Anordnungen als digitale Verzögerungsleitungen verwendet. Um ein digitales Wort, bestehend aus m-Bits, um eine Pulswiederholungsperiode zu verzögern, benötigt man m-Schieberegister zu je N-Bits, die alle mit der Taktfrequenz $f_c$ betrieben werden.

Im oben beschriebenen Ausführungsbeispiel liegen die Eingangssignale A, B bzw. A', B' des Diskriminators 73 in digitaler Form vor. Bei einer anderen Struktur des Ultraschall-Pulsechogeräts könnten diese Signale auch in analoger Form vorliegen. In beiden Fällen können die Eingangssignale A, B bzw. A', B' z.B. binär oder ternär codiert aus den vorliegenden Signalen in an sich bekannter Weise, wie folgt abgeleitet werden:

– Binäre Signale A bzw. B können aus entsprechenden Analogsignalen, z.B. mit Spannungskomparatoren wie LM 311 von National Semiconductors Ltd. erzeugt werden; aus einem in "two's complement" codierten Digitalsignal nimmt man einfach das Vorzeichenbit. Wie in Fig. 16 gezeigt, werden die so erzeugten binär codierten Signale A und B mit einer geeigneten Schieberegister-Anordnung 24 (diese enthält die Schieberegister 71, 72 in Fig. 7), die eine Speicherkapazität von 2 × N Bits hat, je um eine Pulswiederholungsperiode verzögert, um die verzögerten, binär codierten Signale A' bzw. B' zu erzeugen.

– Ternäre Signale A bzw. B können ebenfalls aus entsprechenden Analogsignalen mit je zwei Spannungskomparatoren abgeleitet werden. Zur Erzeugung ternär codierter Signale A bzw. B aus einem digitalen Signal kann man zwei digitale Komparatoren (aufgebaut mit der Schaltung SN 7485 von Texas Instruments) verwenden. Die ternär codierten Signale A und B werden mit der Schieberegister-Anordnung 24 verzögert, die dafür eine Speicherkapazität von 4 × N Bits hat, um die verzögerten, binär codierten Signale A' bzw. B' zu erzeugen.

Im folgenden wird davon ausgegangen, dass die Eingangssignale A, B und A', B' in der für ihre Verarbeitung im Diskriminator 73 geeigneten Form, d.h. z.B. binär oder ternär codiert, dessen Eingängen zugeführt werden. Im allgemeinen ist die im Diskriminator 73 durchgeführte Signalverarbeitung auch mit Eingangssignalen höherer Auflösung möglich und für manche Anwendungen möglicherweise sinnvoll; die erforderliche Schaltung wird jedoch entsprechend aufwendiger.

Diskriminator für binär codierte Eingangssignale

Die Eingangssignale A, B bzw. A', B' sind je ein digitales 1-Bit-Signal, das die Vorzeichen-Information des Signals trägt, von dem es abgeleitet wurde. Diese Eingangssignale werden dem Phasendifferenzdetektor 11 zugeführt, der im vorliegenden Fall aus einer kombinatorischen Schaltung besteht, die die Signale A, B, A', B' verknüpft, um zwei Ausgangssignale Re bzw. Im zu erzeugen, welche gemeinsam die Information über die Phasendifferenz zwischen den durch A, B bzw. A', B' gebildeten Vektoren tragen, indem Re und Im einen Vektor definieren, dessen Phase (bezogen auf die Re-Achse) der oben erwähnten Phasendifferenz entspricht. Infolge der groben Quantisierung von A, B bzw. A', B' sind nur 4 verschiedene Zahlenpaare Re, Im möglich.

Fig. 17 zeigt die Wahrheitstabelle der mit der oben erwähnten kombinatorischen Schaltung durchgeführten Verknüpfung. In dieser Tabelle sind die binär "two's complement" codierten Äquivalente in Klammern angegeben.

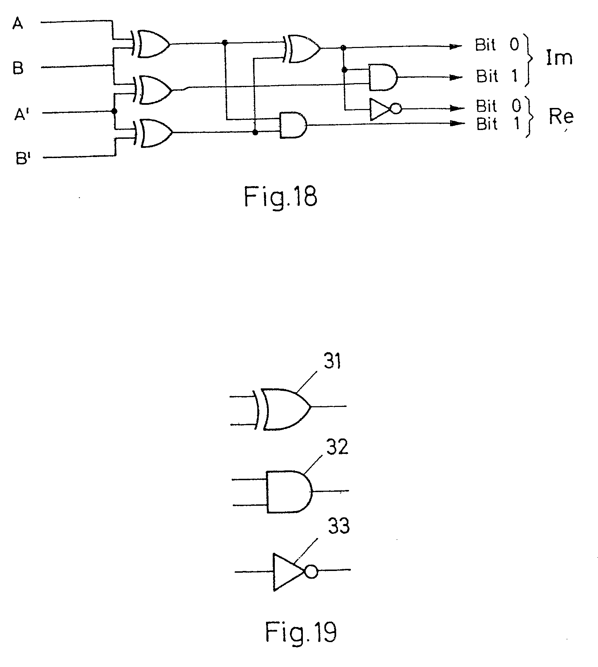

Die Fig. 18 zeigt die Realisierung der Verknüpfung gemäss der Wahrheitstabelle in Fig. 17 mit Gattern. Die Fig. 19 zeigt die in Fig. 18 verwendeten Symbole: ein Exklusiv-ODER Gatter 31 (die integrierte Schaltung SN 7486 von Texas Instruments enthält vier solcher Gatter), ein UND-Gatter 32 (die integrierte Schaltung SN 7408 von Texas Instruments enthält vier solcher Gatter) und einen Inverter 33 (die integrierte Schaltung SN 7404 von Texas Instruments enthält sechs solcher Inverter).

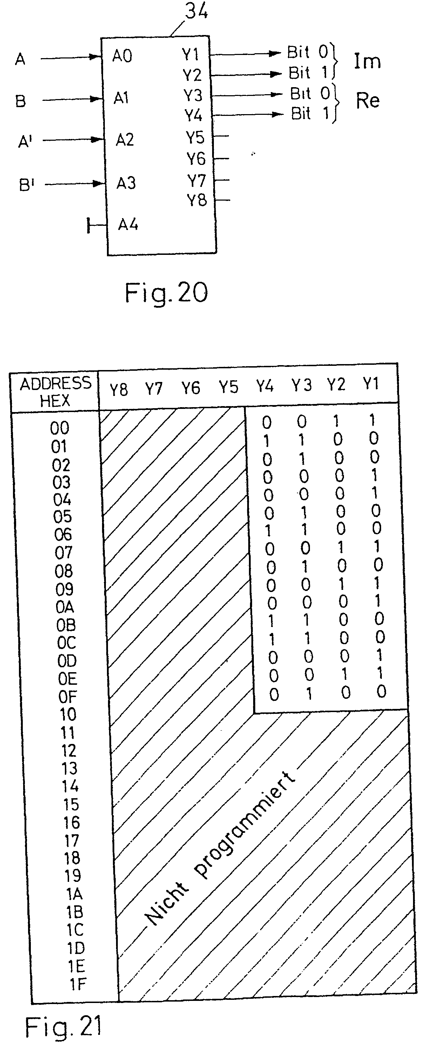

Wie in Fig. 20 gezeigt kann die Realisierung der Verknüpfung gemäss der Wahrheitstabelle in Fig. 17 auch mit einem PROM 34, z.B. mit der integrierten Schaltung SN 74 S288 von Texas Instruments, realisiert werden. Die dafür erforderliche Programmierung des PROMS ist aus der Fig. 21 ersichtlich.

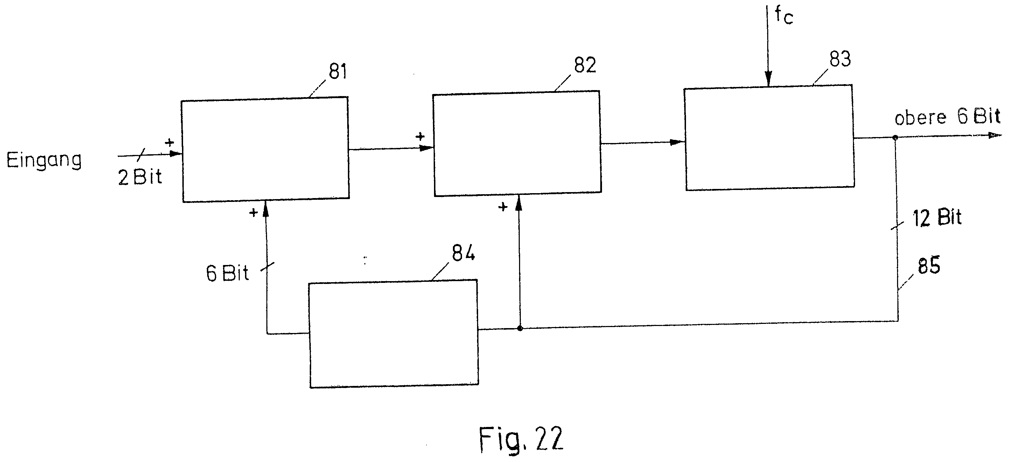

Die mit dem Phasendifferenzdetektor 11 erzeugten Signale Re und Im werden ausgemittelt. Diese Ausmittelung kann kontinuierlich durch ein periodisches Tiefpassfilter vorgenommen werden oder durch Integration einer gewissen Zahl aufeinanderfolgender Pulse mit anschliessendem Löschen aller für die Integration verwendeten Speicher. Fig. 22 zeigt als Beispiel der Ausmit-telungsschaltung einen abklingenden Sweep Inte-grator, der einem n-kanaligen Tiefpassfilter erster Ordnung entspricht. Dieser Sweep Integrator enthält einen 6-Bit-Addierer 81 (hergestellt z.B. mit zwei integrierten Schaltungen SN 7483 von Texas Instruments), einen 12-Bit-Addierer 82 (hergestellt z.B. mit drei integrierten Schaltungen SN 74 283 von Texas Instruments), ein 12 × N Bit Schiebe-register 83, das, getaktet durch die Taktfrequenz $f_c$, als Verzögerungsleitung arbeitet (siehe obige Beschreibung anhand der Fig. 14 und 15), und eine Rückkopplungsstufe 84.

Das 2-Bit Eingangssignal (Re oder Im) wird dem Addierer 81 zugeführt. Das 12-Bit Ausgangssignal 85 des Schieberegisters 83 wird in der Rückkopplungsstufe mit einer Konstanten K multipliziert und das Produkt zum Addierer 81 zurückgeführt. Die Zeitkonstante des in Fig. 22 gezeigten Tief-passfilters beträgt $\frac{1}{1-K}$ Pulswiederholungsperio-den. Da im vorliegenden Beispiel eine Zeitkon-stante von 64 Pulswiederholungsperioden be-nötigt wird, ist K = 63/64 zu wählen. Die Durchführung der Multiplikation des 12-Bit Ausgangssignals 85 mit diesem Wert von K wird vereinfacht, wenn man anstelle des Produkts $(63/64) \cdot x$ die Differenz $x - \frac{1}{64}x$ bildet, wobei $(63/64) \cdot x = x - \frac{1}{64}x$. Die Multiplikation mit dem Faktor $-1/64$ wird mit der Stufe 84 und dem Addierer 81 durch 6-Stellen Bitverschiebung, gefolgt von der Bildung des "two's complement" mittels bitweiser Inversion und Addition von 1 am untersten "Carry"-Eingang des Addierers 81 durchgeführt. Bei einem Ausgangssignal von 6 Bit-Auflösung muss die Verzögerungsleitung mit 12 Bit arbeiten, damit ein Abklingen auf 0 möglich ist.

Die von den Sweep Integrators 12, 13 abgegebenen, ausgemittelten Signale $\overline{Re}$ und $\overline{Im}$ werden in der Recheneinheit 14 ausgewertet. Die Recheneinheit 14 kann am einfachsten unter Verwendung von Festwertspeichern (ROM) realisiert werden.

Die zu realisierende Berechnung lautet für die Geschwindigkeit:

$$V = \overline{\Delta\varphi} = \begin{cases} \dfrac{\overline{Im}}{|\overline{Re}| + |\overline{Im}|} & \text{für Re} \geq 0 \\[3mm] 2 \cdot \text{signum}(\overline{Im}) - \dfrac{\overline{Im}}{|\overline{Re}| + |\overline{Im}|} & \text{für Re} < 0 \end{cases}$$

Die Berechnung von $\overline{\Delta\varphi}$ erfolgt durch

$$|\overline{\Delta\varphi}| = \sqrt{|\overline{Re}|^2 + |\overline{Im}|^2}$$

Während die Geschwindigkeit mit 8 Bit-Auflösung berechnet werden sollte, reicht für deren Betrag eine Auflösung mit 4 Bit aus.

Als Ausführungsbeispiel wird nachstehend der Fall beschrieben, bei dem der Betrag der Geschwindigkeit einen bestimmten Grenzwert überschreiten muss, damit die Rechnung gültig ist. Andernfalls wird der Ausgangswert Null gesetzt. Die gesamte Auswertung von $\overline{Re}$ und $\overline{Im}$ lautet also wie folgt:

Eingangssignal:    $\overline{Re}$: 6 Bit two's complement code entsprechend dem Zahlenbereich $-32 \ldots +31$
$\overline{Im}$: wie $\overline{Re}$

Ausgangssignal:    V: 8 Bit two's complement code entsprechend dem Zahlenbereich $-127 \ldots +127$

Das Ausgangssignal V muss kalibriert werden. Für 4 MHz Ultraschallfrequenz und $f_R = 10$ kHz wird der Bereich $\pm 127$ äquivalent $\pm 1$ m/s.

Zur Erzeugung eines Strömungsprofils auf einem Beobachtungsmonitor muss das Signal V mittels Digital-Analog-Konverter ins Ausgangssignal 59 (Fig. 7) umgewandelt werden.

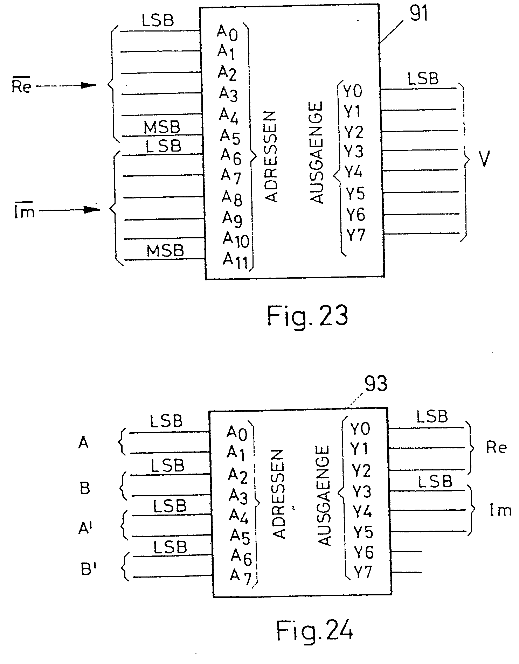

Fig. 23 zeigt, wie ein ROM (Read Only Memory) 91 als Recheneinheit 14 (siehe Fig. 12) eingesetzt wird. Das ROM 91, das 4096 Worte à 8 Bit enthalten kann, setzt sich z.B. aus vier integrierten Schaltungen 4 × 2708 von Intel zusammen. Die Leitungen mit dem LSB ("least significant bit") bzw. MSB ("most significant bit") sind in Fig. 23 mit diesen Abkürzungen markiert.

Die Programmierung des ROM's 91 erfolgt nach untenstehender Formel:

Wenn $\sqrt{\overline{Im^2} + \overline{Re^2}} < 15$    dann ist V = 0;

$$\text{sonst } v = \begin{cases} 127 \cdot \dfrac{\overline{Im}}{\overline{Re} + \overline{Im}} & \text{für Re} \leq 0 \\[3mm] 127 \cdot \left[ 2 \cdot \text{signum}(\overline{Im}) - \dfrac{\overline{Im}}{|\overline{Re}| + |\overline{Im}|} \right] & \text{für Re} < 0 \end{cases}$$

Die Codierung von V erfolgt durch 8 Bit two's complement code.

Diskriminator für ternär codierte Eingangssignale

A, B bzw. A', B' sind je ein digitales 2-Bit-Signal, welches drei Zustände annehmen kann für die Fälle, dass das Eingangssignal in einem kleinen Bereich um 0 herum, über der oberen Grenze dieses Bereiches oder unter der unteren Grenze liegt. Diese drei Fälle werden durch die Zahlen 0 (binär 00); +1 (binär 01) bzw. −1 (binär 11) codiert.

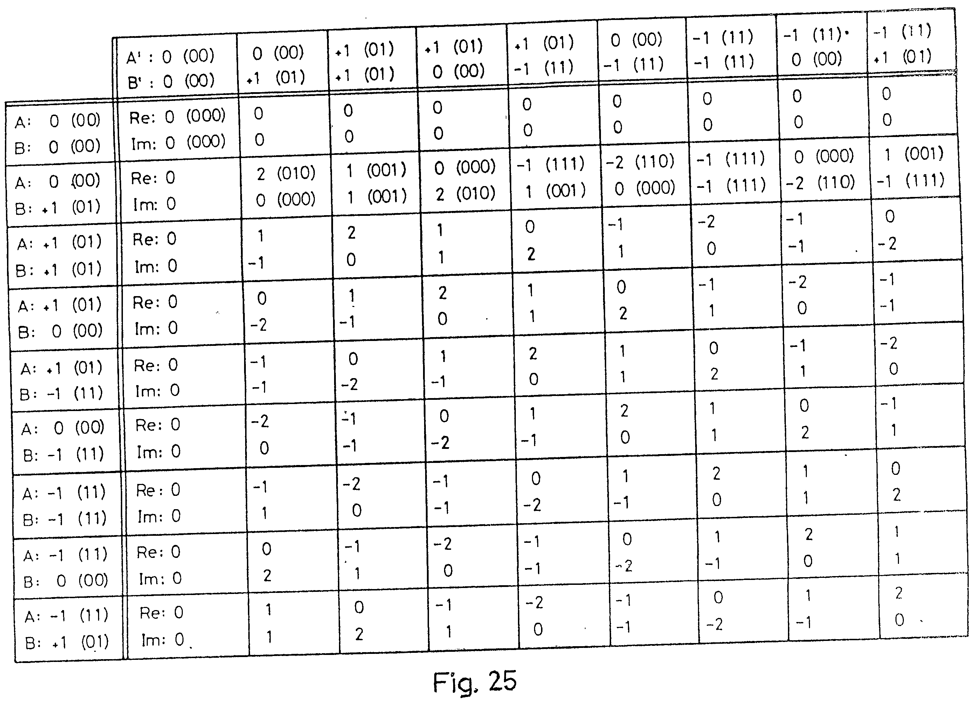

In diesem Fall hat das Schieberegister 24 in Fig. 16 eine Kapazität von 4 × N Bits und die Ausgangssignale Re und Im des Phasendifferenzdetektors 11 weisen je 3 Bit auf. Die Realisierung der Schaltung zur Verknüpfung von A, B und A', B' erfolgt am zweckmässigsten durch einen Festwertspeicher 93. Man benötigt 8 Adresseingänge und 6 Datenausgänge. Verwendet wird beispielsweise das PROM SN 74 S470 von Texas Instruments. Fig. 24 zeigt die entsprechende Beschaltung. Fig. 25 zeigt die Funktionstabelle, aus welcher die Programmierung des PROM's leicht abgeleitet werden kann. In dieser Tabelle ist das binär "two's complement" codierte Äquivalent jeweils in Klammern angegeben.

Die Ausmittelung von Re bzw. Im erfolgt mit der Schaltung nach Fig. 22 mit dem Unterschied, dass das Eingangssignal 3 Bit Auflösung besitzt.

Die Recheneinheit 14 ist identisch mit derjenigen des Diskriminators für binärcodiertes Eingangssignal (siehe Fig. 23).

Der Vorteil des soeben beschriebenen Verfahrens mit ternärcodierten Eingangssignalen A, B bzw. A', B' liegt darin, dass im Falle wo kein Dopplersignal vorhanden ist, der Betrag

$$\sqrt{\overline{Im^2} + \overline{Re^2}}$$

zuverlässig gegen 0 strebt, da die übrigbleibenden kleinen Rauschsignale oft in die schmale Nullpunkt-Zone fallen. In diesem Falle wird A bzw. B jeweils 0 und nach der Verknüpfung auch Re und Im. Für starke Dopplersignale hingegen erbringt dieses Verfahren praktisch keine Vorteile gegenüber dem Verfahren mit binärcodierten Eingangssignalen.

### Verbesserung der Linearität

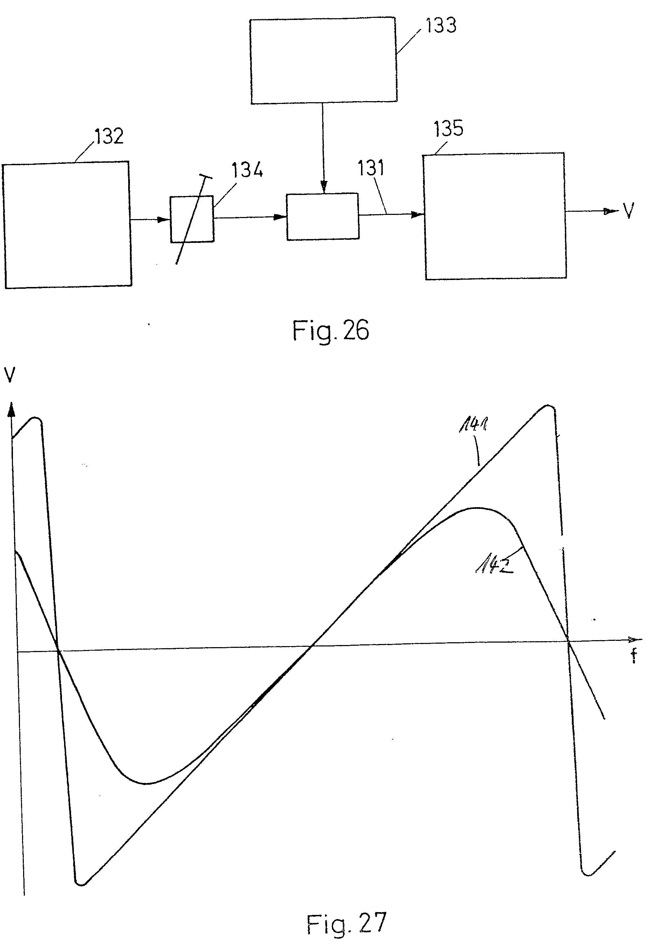

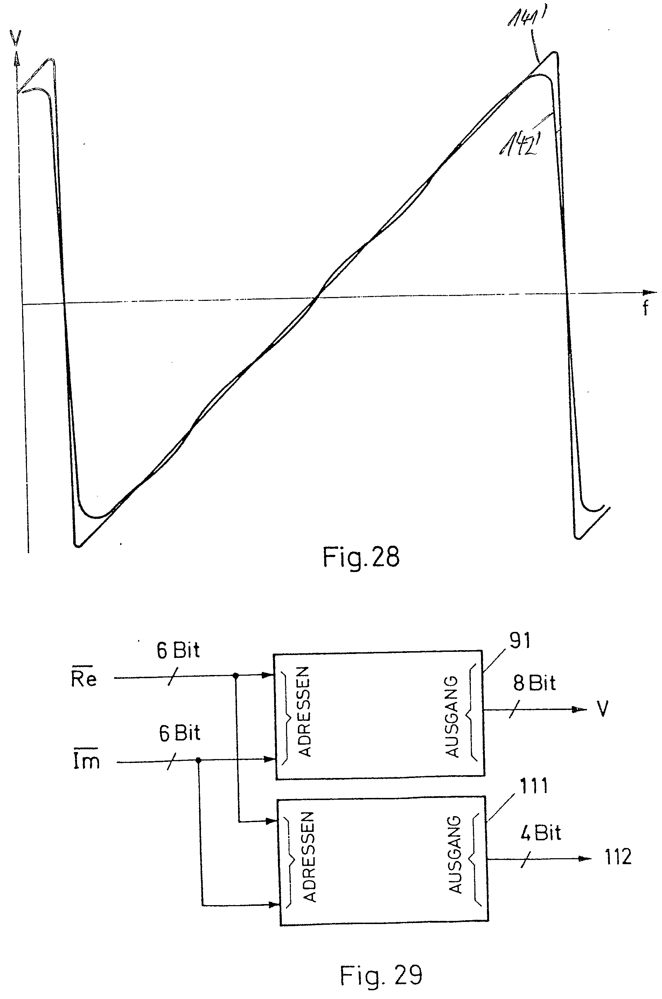

Die mit dem oben beschriebenen Diskriminator 73 für binärcodierte Eingangssignale erzielte Verbesserung der Linearität der Messung ist durch Gegenüberstellung der in den Fig. 27 und 28 gezeigten Messergebnisse feststellbar. Diese Ergebnisse sind mit der Messanordnung nach Fig. 26 unter gleichen Bedingungen ermittelt worden. Wie dort schematisch gezeigt, wird ein Eingangssignal 131, das dem Signal 55 in Fig. 7 entspricht, durch Überlagerung der Ausgangssignale eines Signalgenerators 132 mit variabler Frequenz und einer Rauschquelle 133 erzeugt. Dabei kann das Signal/Rauschenverhältnis mit einem variablen Dämpfungsglied 134 eingestellt werden. Das Eingangssignal 131 wird dem verwendeten Dopplerfrequenzdetektor 135 zugeführt.

Fig. 27 zeigt die Geschwindigkeits-Frequenz-Charakteristika 141 des Ausgangssignals 59 mit einem Signal/Rauschen-Verhältnis von 26 dB und den gemessenen Verlauf 142 dieser Charakteristik, mit einem Signal/Rauschen-Verhältnis von 6 dB, wenn ein "zero crossing counter" nach McLeod, "A multiple gate pulse Doppler Flowmeter 1971 IEEE Ultrasonics Symposium", Miami Beach, Florida, als Dopplerfrequenzdetektor verwendet wird.

Fig. 28 zeigt die mit den gleichen Werten (26 dB bzw. 6 dB) des Signal/Rauschen-Verhältnisses gemessenen Charakteristiken, 141', 142', wenn die Anordnung gemäss der Fig. 7 mit dem oben beschriebenen Diskriminator 73 als Dopplerfrequenzdetektor verwendet wird. Die mit dieser letzten Anordnung erreichte, relative Unabhängigkeit vom Signal/Rauschen-Verhältnis ist offensichtlich.

### Ausgestaltung des Diskriminators 73

Fig. 29 zeigt eine Ausgestaltung der Recheneinheit 14 (siehe Fig. 12) des Diskriminators 73. Es ist mit dieser Ausgestaltung möglich eine Anzeige zu erzeugen, aus der auf die Turbulenz der gemessenen Strömung geschlossen werden kann.

Wie oben bereits erwähnt, werden den Eingängen der Recheneinheit 14 Signale $\overline{Re}$ und $\overline{Im}$ zugeführt, die den mittleren Wert der einzeln durch Re und Im definierten Phasendifferenzvektoren entsprechen.

Bei einwandfreier Signalqualität und stationärer Strömung sind die Ergebnisse Re, Im der Verknüpfung der Eingangssignale A, B, A', B' konstant, so dass der Betrag des ausgemittelten Phasendifferenzvektors gleich der Summe der Beträge der einzelnen Phasendifferenzvektoren ist. Dies gilt dann

$$\sum_{i=0}^{n} (Re_i, Im_i) = n \cdot Re, \, n \cdot Im$$

und $\sqrt{\overline{Re^2} + \overline{Im^2}} = n \cdot \sqrt{Re^2 + Im^2}$

Weisen jedoch die einzelnen Phasendifferenzvektoren Re, Im eine statische Schwankung auf, so ist

$$\sqrt{\overline{Re^2} + \overline{Im^2}} < n \cdot \sqrt{Re^2 + Im^2}$$

Die statische Schwankung kann zwei Ursachen haben:
1) schlechter Geräuschspannungsabstand des Signals
2) turbulente Strömung

Kann die Ursache 1) ausgeschlossen werden (z.B. durch hohe Sendeleistung und/oder hohe Empfindlichkeit des Empfängers), so bleibt nur die Turbulenz der Strömung als mögliche Ursache der statischen Schwankung.

Im Falle mit binärcodierten Eingangssignalen A, B, A', B' gilt $\sqrt{Re^2 + Im^2} = 1$ für alle Re-, Im-Kombinationen. (Bei ternärer Codierung gilt dies nur annäherungsweise.)

Somit gilt

$$\frac{\sqrt{\overline{Re^2} + \overline{Im^2}}}{n} \leq 1$$

Wie in Fig. 29 gezeigt, enthält die soeben erwähnte Ausgestaltung der Recheneinheit 14 den bereits in Fig. 23 gezeigten Festwertspeicher 91, mit dem das Ausgangssignal V erzeugt wird, und einen Festwertspeicher 111, mit dem ein Ausgangssignal 112 erzeugt wird, das proportional zu

$$\frac{\sqrt{\overline{Re^2} + \overline{Im^2}}}{n}$$

ist, wobei der Proportionalitätsfaktor z.B. 15 beträgt.

Die Anzahl ausgemittelter Messwerte (Re, Im) muss bekannt sein und sei gleich n.

Wenn die Signale $\overline{Re}$ und $\overline{Im}$ mit dem in Fig. 22 gezeigten Tiefpassfilter erzeugt werden, lässt sich eine Zahl $n = \dfrac{1}{1-K}$ berechnen, welche die Anzahl ausgemittelter Messwerte von Re bzw. Im ausdrückt, wobei K der Multiplikator in der Rückkopplung des Tiefpassfilters (Ausmittelungsschaltung) ist. Im Beispiel der Fig. 22 ist $K = \dfrac{63}{64}$ und somit n = 64.

Fig. 30 zeigt eine schematische Darstellung der genannten Anzeige, die durch Verwendung der in Fig. 29 gezeigten Ausgestaltung der Recheneinheit 14 und eines Bildschirms 121 erzeugt werden kann. Aus dieser Anzeige sind ein Geschwindigkeitsprofil 122 der gemessenen Strömung und eine Anzeige 123 der Turbulenz dieser Strömung ersichtlich. Das Profil 122 entspricht dem Verlauf des Ausgangssignals 59 des Diskriminators 73 und wird auf kalibrierten Achsen V–X dargestellt, wobei V die Punktgeschwindigkeit und X die Entfernung zwischen dem Ultraschallwandler und dem Punkt, bei dem die Punktgeschwindigkeit gemessen wird, bedeuten. Die Anzeige 123 entspricht dem Verlauf des Signals 112 (Fig. 29) und hat an sich zwei verschiedene Bedeutungen, je nachdem, ob man die Anzeige betreffend Punkten ausserhalb oder innerhalb der gemessenen Strömung beachtet. Für Punkte ausserhalb der gemessenen Strömung, d.h. für die Bereiche des Profils 122, bei denen V = 0, bedeuten sichtbare Werte der Anzeige 123, je nach ihrer Grösse, dass die Eingangssignale A, B, A', B' ein gutes (Anzeige 123 tendiert gegen 1) oder mehr oder weniger schlechtes (Anzeige 123 tendiert gegen Null) Signal/Rauschen-Verhältnis aufweisen. Bei Verwendung eines Festzeichenlöschers, wie oben beschrieben, wird die Anzeige 123 für Punkte ausserhalb der Strömung stets gegen 0 tendieren, da die Signale A, B bzw. A', B' praktisch nur aus Rauschen bestehen. Für Punkte innerhalb der gemessenen Strömung ist die Anzeige 123 ein Indikator des Turbulenzgrades der Strömung in untersuchtem Querschnitt derselben. Wie in Fig. 30 gezeigt, ist neben der Anzeige 123 eine vertikale Achse vorgesehen, aus der die für die Interpretation der Anzeige 123 interessierende Punkte 0 und 1 ersichtlich sind. Liegt die Anzeige 123 nahe dem Wert 1, so kann die Strömung als laminar bezeichnet werden. Andernfalls wird man sie als turbulent bezeichnen.

Allgemeine Ausführungsform des Phasendifferenzdetektors 11

Wie oben bereits erwähnt, dient der Phasendifferenzdetektor 11 des Diskriminators 73 (Fig. 12) dazu, mit den Eingangssignalen A, B bzw. A', B' je eine komplexe Zahl

$$Z_1 = A + j\,B$$
$$Z_2 = A' + j\,B'$$

zu bilden, durch Bildung des Quotienten $Z_1/Z_2$ dieser beiden Zahlen eine komplexe Zahl

$$Re + j\;Im = \frac{A+jB}{A'+jB'}$$

zu bilden, deren Komponenten Re und Im mit den Komponenten des Phasendifferenzvektors $\overrightarrow{\Delta\varphi}$ identisch sind, und Ausgangssignale zu erzeugen, die den Komponenten Re bzw. Im entsprechen.

Wie in Fig. 31 gezeigt, lässt sich ein derartiger Phasendifferenzdetektor – für den Fall, dass A, B, A', B' Binärzahlen mit i-Bits sind – mit einem PROM 141 realisieren, in dem die Werte von Re und Im für alle möglichen Kombinationen von A, B, A', B' gespeichert sind. Besitzen diese Eingangssignale je i-Bits und die Ausgangssignale (Re und Im) je p-Bits, so gibt es $2^{4i}$ Lösungswerte für Im bzw. Re und das erforderliche PROM-Format ist $2^{4i} \times 2p$.

Die Ausführung des Phasendifferenzdetektors mit einem PROM für binär- bzw. ternärcodierte Eingangssignale A, B, A', B' ist oben anhand der Fig. 20, 21 bzw. 24, 25 beschrieben.

Für binärcodierte Eingangssignale ist i = 1, p = 2 und das erforderliche PROM-Format 16 × 4 Bit.

Für ternärcodierte Eingangssignale ist i = 2, p = 3 und das erforderliche PROM-Format 256 × 6 Bit.

Für i = 4 und p = 5 ist das erforderliche PROM-Format $2^{16} \times 10$ Bit = 64k × 10 Bit. Da die grössten gegenwärtig erhältlichen PROM eine Kapazität von 8k × 8 Bit hat, benötigt man 10 Stück davon. Um diesen Aufwand zu reduzieren, ist es vorteilhaft die komplexe Zahl Re + j Im wie folgt auszudrücken:

$$Re + jIm = \frac{A+jB}{A'+jB'} \cdot \frac{A'-jB'}{A'-jB'} = \frac{A \cdot A' + B \cdot B' + j(BA' - AB')}{A'^2 + B'^2}$$

$$Re = \frac{A \cdot A' + B \cdot B'}{A'^2 + B'^2} = \frac{A}{A' + B'^2/A'} + \frac{B}{A'^2/B' + B'}$$

$$Im = \frac{B \cdot A' - A \cdot B'}{A'^2 + B'^2} = \frac{B}{A' + B'^2/A'} - \frac{A}{A' +^2/B' + B'}$$

Durch diese Umformung ist es nun möglich die Ausgangssignale Re und Im mit der in Fig. 32 gezeigten Phasendifferenzdetektor-Schaltung zu erzeugen. Diese besteht aus 5 PROM 151–155, einem Inverter 156 und zwei Addierern 157, 158.

Mit dem PROM 151 werden Ausgangssignale erzeugt, die den Werten

$$K1 = A' + B'^2/A'$$
$$K2 = A'^2/B' + B'$$

entsprechen. Wenn die Signale A', B' und $K_1$, $K_2$

eine i-Bit-Auflösung haben, muss das PROM 151 das Format $2^{2i} \times 2i$ haben.

Mit den vier PROM 152–152 mit je einem Format $2^{2i} \times i$ werden Ausgangssignale gebildet, die den Quotienten

$$Q_1 = A/K1$$
$$Q_2 = B/K2$$
$$Q_3 = B/K1$$
$$Q_4 = A/K2$$

entsprechen.

Mit dem Inverter 156 und den Addieren 157, 158 werden anschliessend die Ausgangssignale

$$Re = Q_1 + Q_2$$
$$Im = Q_3 - Q_4$$

gebildet.

Für das Beispiel mit $i \cdot 4$ und $p = 5$ benötigt man mit der Schaltung gemäss Fig. 32:

1 PROM 256 × 8 Bit
4 PROM 256 × 4 Bit
1 Inverter-IC z.B. SN 7404 (von Texas Instruments)
2 Addierer z.B. SN 74 283 (von Texas Instruments)

d.h. der Speicherbedarf ist gegenüber der direkten Berechnung von Re und Im mit der Anordnung gemäss Fig. 31 von 64 kbit auf 6 kbit reduziert.

**Patentansprüche**

1. Verfahren zur Bestimmung von Strömungsgeschwindigkeiten durch Messung der Phasendifferenz zwischen Dopplersignalen, die von Wellenimpulsen abgeleitet sind, die im wesentlichen von einem und demselben reflektierenden Bestandteil der untersuchten Strömung zu zwei verschiedenen Zeitpunkten reflektiert werden, wobei das Intervall zwischen den Zeitpunkten vorbestimmt ist, bei welchem Verfahren von jedem Dopplersignal durch Quadratur-Demodulation ein Paar elektrischer Signale abgeleitet wird, die gemeinsam Information bezüglich der Phase des Dopplersignals tragen, wobei ein erstes und ein zweites Signalpaar (A und B bzw. A' und B') miteinander verknüpft werden, um ein drittes Paar elektrischer Signale (Re, Im) zu erzeugen, die gemeinsam eine Information tragen, die die Phasendifferenz zwischen den Dopplersignalen in einem bestimmten Bereich eindeutig definiert, dadurch gekennzeichnet, dass die Verknüpfung des ersten und des zweiten Signalpaars so durchgeführt wird, dass die Signale vom dritten Signalpaar Werte annehmen können, die 0, +1 und −1 entsprechen, wodurch der Phasenwinkel des durch das dritte Signalpaar definierten Vektors die Werte 0°, 45°, 90°, 135°, 180°, 225°, 270° und 315° annehmen kann, und dass aus mehreren dritten Signalpaaren Mittelwert-Signale (Re, Im) gebildet werden, die je dem Mittelwert eines der Signale vom dritten Signalpaar entsprechen, und von den Mittelwert-Signalen ein Ausgangssignal abgeleitet wird, das dem Mittelwert $(\overline{\Delta\varphi})$ der Phasendifferenz zwischen den Dopplersignalen entspricht.

2. Vorrichtung zur Durchführung des Verfahrens gemäss Anspruch 1, mit einer Einrichtung (41) zur Beaufschlagung der zu untersuchenden Strömung mit Wellenimpulsen, mit einem Empfänger (42) für die von Bestandteilen der Strömung reflektierten Dopplersignale, mit einem Quadraturdemodulator (43) zur Ableitung eines Signalpaars aus jedem empfangenen Dopplersignal, mit einer Schaltung (11) zur Verknüpfung eines ersten solchen mit einem zweiten solchen Signalpaar, um ein drittes Paar elektrischer Signale (Re, Im) zu erzeugen, die gemeinsam eine Information tragen, die die Phasendifferenz zwischen den Dopplersignalen in einem bestimmten Bereich eindeutig definiert, dadurch gekennzeichnet, dass die Schaltung (11) so ausgebildet ist, dass die Signale vom dritten Signalpaar Werte annehmen können, die 0, +1 und −1 entsprechen, wodurch der Phasenwinkel des durch das dritte Signalpaar definierten Vektors die Werte 0°, 45°, 90°, 135°, 180°, 225°, 270° und 315° annehmen kann, und dass die Schaltung Mittel (12, 13) zur Bildung von Mittelwert-Signalen $(\overline{Re}, \overline{Im})$ aus mehreren dritten Signalpaaren aufweist, wobei die Mittelwertsignale je dem Mittelwert eines der Signale vom dritten Signalpaar entsprechen, sowie eine Recheneinheit (14) zur Verarbeitung der Mittelwert-Signale, mit der ein Ausgangssignal (59) erzeugbar ist, das dem Mittelwert der Phasendifferenz zwischen den Dopplersignalen entspricht.

3. Vorrichtung nach Anspruch 2, dadurch gekennzeichnet, dass die Recheneinheit (14) Mittel zur Berechnung des Betrages der Summe der Vektoren, die durch dritte Signalpaare (Re, Im) für eine Mehrzahl von Dopplersignalen definiert werden, und Mittel zur Unterdrückung des Ausgangssignals der Recheneinheit in Abhängigkeit vom Wert des berechneten Betrages enthält.

4. Vorrichtung nach Anspruch 2, dadurch gekennzeichnet, dass die Recheneinheit Mittel zur Erzeugung eines Ausgangssignals enthält, das proportional zu

$$\frac{\sqrt{\overline{Re}^2 + \overline{Im}^2}}{n}$$

ist, worin $\overline{Re}$ und $\overline{Im}$ die Mittelwerte des dritten Signalpaars sind und n die Anzahl der ausgemittelten dritten Signalpaare (Re, Im) ist.

5. Verwendung des Verfahrens gemäss Anspruch 1 in einem Ultraschall-Diagnostikgerät, das zur Bestimmung eines Geschwindigkeitsprofils einer Strömung dient.

6. Verwendung nach Anspruch 5, dadurch gekennzeichnet, dass die Strömung die Blutströmung in einem Blutgefäss ist.

**Claims**

1. A method of determining flow velocities by measuring the phase difference between Doppler signals derived from wave pulses which are reflected by substantially the same reflecting component of the flow under examination, at two different times, the interval between the times being predetermined, in which method a pair of electric signals is derived from each Doppler signal by quadrature demodulation, the signals jointly bearing information relating to the phase of the Doppler signal, a first and a second signal pair (A and B & A' and B') being combined to produce a third pair of electric signals (Re, Im) which jointly bear information which unequivocally defines the phase difference between the Doppler signals in a specific scope, characterised in that the combination of the first and second pairs of signals is so carried out that the signals of the third pair can assume values corresponding to 0, + 1 and − 1, so that the phase angle of the vector defined by the third signal pair can assume the values 0°, 45°, 90°, 135°, 180°, 225°, 270° and 315°, and that from a plurality of third signal pairs mean-value signals are formed (Re, Im) each corresponding to the mean value of one of the signals of the third signal pair and an output signal is derived from the mean value signals, said output signal corresponding to the mean value ($\overline{\Delta\varphi}$) of the phase difference between the Doppler signals.

2. A device for performing the method according to claim 1, comprising means (41) for irradiating the flow under examination with wave pulses, a receiver (42) for the Doppler signals reflected by components of the flow, a quadrature demodulator (43) for deriving a signal pair from each received Doppler signal, a circuit (11) for combining a first such signal pair with a second such signal pair in order to produce a third pair of electric signals (Re, Im) which jointly bear information unequivocally defining the phase difference between the Doppler signals in a specific scope, characterised in that the circuit (11) is so constructed that the signals of the third signal pair can assume values corresponding to 0, + 1 and − 1, so that the phase angle of the vector defined by the third signal pair can assume the values 0°, 45°, 90°, 135°, 180°, 225°, 270° and 315°, and the circuit contains means (12, 13) for forming mean value signals ($\overline{Re}$, $\overline{Im}$) from a plurality of third signal pairs, the mean value signals each corresponding to the mean value of one of the signals of the third signal pair, and a computer unit (14) for processing the mean value signals, to produce an output signal (59) which corresponds to the mean value of the phase difference between the Doppler signals.

3. A device according to claim 2, characterised in that the computer unit (14) contains means for calculating the amount of the sum of the vectors defined by third signal pairs (Re, Im) for a plurality of Doppler signals, and means for suppressing the output signal of the computer unit in dependence on the value of the calculated amount.

4. A device according to claim 2, characterised in that the computer unit contains means for producing an output signal proportional to

$$\frac{\sqrt{\overline{Re}^2 + \overline{Im}^2}}{n}$$

where $\overline{Re}$ and $\overline{Im}$ are the mean values of the third signal pair and n is the number of averaged third signal pairs (Re, Im).

5. Use of the method according to claim 1 in an ultrasonic diagnostic device for determining a velocity profile of a flow.

6. Use according to claim 5, characterised in that the flow is the blood flow in a blood vessel.

**Revendications**

1. Procédé pour la détermination de vitesses d'écoulement par mesure de la différence de phase entre des signaux Doppler dérivés d'impulsions d'onde, qui sont réfléchies essentiellement par une même composante réfléchissante de l'écoulement étudié, à deux instants différents dont l'intervalle est prédéterminé, et selon lequel à partir de chaque signal Doppler est dérivé par démodulation en quadrature, un couple de signaux électriques portant l'information commune concernant la phase du signal Doppler, et selon lequel un premier et un second couple de signaux (A et B ou A' et B') sont combinés entre eux pour former un troisième couple de signaux électriques (Re, Im), qui portent en commun une information définissant nettement la différence de phase entre les signaux Doppler dans une gamme déterminée, caractérisé en ce que la combinaison du premier et du second couples de signaux est réalisée de telle manière que les signaux du troisième couple de signaux peuvent prendre des valeurs qui correspondent à 0, + 1 et − 1, ce qui a pour effet que l'angle de phase du vecteur défini par le troisième couple de signaux peut prendre les valeurs 0°, 45°, 90°, 135°, 180°, 225°, 270° et 315°, et qu'à partir de plusieurs troisièmes couples de signaux, on forme des signaux de valeur moyenne (Re, Im) qui correspondent respectivement à la valeur moyenne de l'un des signaux du troisième couple de signaux et on tire, des signaux de valeur moyenne le signal de sortie qui correspond à la valeur moyenne ($\overline{\Delta\varphi}$) de la différence de phase entre les signaux Doppler.

2. Dispositif pour la mise en œuvre du procédé selon la revendication 1, à l'aide d'un dispositif (41) servant à charger l'écoulement devant être étudié par des impulsions d'onde, un récepteur (42) pour les signaux Doppler réfléchis par des composantes de l'écoulement, un démodulateur en quadrature (43) servant à dériver un couple de signaux à partir de chaque signal de Doppler reçu, un circuit (11) pour combiner un tel premier couple de signaux avec un tel second couple de signaux pour produire un troisième couple de signaux électriques (Re, Im), qui portent en commun une information définissant de façon nette la différence de phase entre les signaux Doppler dans une gamme déterminée, caractérisé en ce que le circuit (11) est constitué de telle manière que les signaux du troisième couple de signaux peuvent prendre des valeurs qui correspondent à 0, + 1 et − 1, ce qui a pour effet que l'angle de phase du vecteur défini par le troisième couple de signaux peut prendre les valeurs 0°, 45°, 90°, 135°, 180°, 225°, 270° et 315°, et que le circuit comporte des moyens (12, 13) servant à former des signaux de valeur moyenne ($\overline{Re}$, $\overline{Im}$) à partir de plusieurs troisièmes couples de signaux, lesdits signaux de valeur moyenne correspondant respectivement à la valeur moyenne de l'un des signaux du troisième couple de signaux, ainsi qu'une unité de calcul (14) servant à traiter les signaux de valeur moyenne et à l'aide duquel peut être produit un signal de sortie (59) qui correspond à la valeur moyenne de la différence de phase entre les signaux Doppler.

3. Dispositif selon la revendication 3, caractérisé en ce que l'unité de calcul (14) contient des moyens servant à calculer la valeur de la somme des vecteurs, qui sont définis par les troisièmes couples de signaux (Re, Im) pour un grand nombre de signaux Doppler, et des moyens servant à supprimer le signal de sortie de l'unité de calcul en fonction de la valeur calculée.

4. Dispositif selon la revendication 2, caractérisé en ce que l'unité de calcul contient des moyens servant à produire un signal de sortie qui est proportionel à

$$\frac{\sqrt{\overline{Re}^2 + \overline{Im}^2}}{n}$$

$\overline{Re}$ et $\overline{Im}$ étant les valeurs moyennes du troisième couple de signaux et n'étant le nombre des troisièmes couples de signaux (Re, Im), dont la moyenne a été réalisée.

5. Mise en œuvre du procédé selon la revendication 1, dans un appareil de diagnostic à ultrasons, qui sert à la détermination d'un profil de vitesses d'un écoulement.

6. Mise en œuvre du procédé selon la revendication 5, caractérisé en ce que l'écoulement est l'écoulement du sang dans un vaisseau sanguin.

Fig. 1

Fig. 2

Fig. 3

0 011 878

Fig. 4

Fig. 5a

Fig. 5b

Fig. 6

0 011 878

Fig.7

19

Fig. 8

Fig. 9

61

0    $t_1$    T

T    $t_2 = t_1 + T$    2T

2T    $t_3 = t_2 + T$    3T

Fig.10

68

90°

69

Fig.11

Fig. 12

Fig.13

Fig.14

Fig.15

Fig. 16

| | A' : +1 (0)<br>B' : +1 (0) | −1 (1)<br>+1 (0) | −1 (1)<br>−1 (1) | +1 (0)<br>−1 (0) |
|---|---|---|---|---|
| A: +1 (0)<br>B: +1 (0) | Re: +1 (01)<br>Im: 0 (00) | 0 (00)<br>+1 (01) | −1 (11)<br>0 (00) | 0 (00)<br>−1 (11) |
| A: −1 (1)<br>B: +1 (0) | Re: 0 (00)<br>Im: −1 (11) | +1 (01)<br>0 (00) | 0 (00)<br>+1 (01) | −1 (11)<br>0 (00) |
| A: −1 (1)<br>B: −1 (1) | Re: −1 (11)<br>Im: 0 (00) | 0 (00)<br>−1 (11) | +1 (01)<br>0 (00) | 0 (00)<br>+1 (01) |
| A: +1 (0)<br>B: −1 (1) | Re: 0 (00)<br>Im: +1 (01) | −1 (11)<br>0 (00) | 0 (00)<br>−1 (11) | +1 (01)<br>0 (00) |

Fig. 17

31

Fig.18

Fig.19

0 011 878

Fig.20

Fig.21

35

Eingang

2 Bit

6 Bit

81

82

f_c

83

obere 6 Bit

12 Bit

84

85

Fig. 22

Fig. 23

Fig. 24

0 011 878

| A': 0 (00) / B': 0 (00) | | 0 (00) / +1 (01) | +1 (01) / +1 (01) | +1 (01) / 0 (00) | +1 (01) / -1 (11) | 0 (00) / -1 (11) | -1 (11) / -1 (11) | -1 (11)· / 0 (00) | -1 (11) / +1 (01) |
|---|---|---|---|---|---|---|---|---|---|
| A: 0 (00) | Re: 0 (000) | 0 | 0 | 0 | 0 | 0 | 0 | 0 | 0 |
| B: 0 (00) | Im: 0 (000) | 0 | 0 | 0 | 0 | 0 | 0 | 0 | 0 |
| A: 0 (00) | Re: 0 | 2 (010) | 1 (001) | 0 (000) | -1 (111) | -2 (110) | -1 (111) | 0 (000) | 1 (001) |
| B: +1 (01) | Im: 0 | 0 (000) | 1 (001) | 2 (010) | 1 (001) | 0 (000) | -1 (111) | -2 (110) | -1 (111) |
| A: +1 (01) | Re: 0 | 1 | 2 | 1 | 0 | -1 | -2 | -1 | 0 |
| B: +1 (01) | Im: 0 | -1 | 0 | 1 | 2 | 1 | 0 | -1 | -2 |
| A: +1 (01) | Re: 0 | 0 | 1 | 2 | 1 | 0 | -1 | -2 | -1 |
| B: 0 (00) | Im: 0 | -2 | -1 | 0 | 1 | 2 | 1 | 0 | -1 |
| A: +1 (01) | Re: 0 | -1 | 0 | 1 | 2 | 1 | 0 | -1 | -2 |
| B: -1 (11) | Im: 0 | -1 | -2 | -1 | 0 | 1 | 2 | 1 | 0 |
| A: 0 (00) | Re: 0 | -2 | -1 | 0 | 1 | 2 | 1 | 0 | -1 |
| B: -1 (11) | Im: 0 | 0 | -1 | -2 | -1 | 0 | 1 | 2 | 1 |
| A: -1 (11) | Re: 0 | -1 | -2 | -1 | 0 | 1 | 2 | 1 | 0 |
| B: -1 (11) | Im: 0 | 1 | 0 | -1 | -2 | -1 | 0 | 1 | 2 |
| A: -1 (11) | Re: 0 | 0 | -1 | -2 | -1 | 0 | 1 | 2 | 1 |
| B: 0 (00) | Im: 0 | 2 | 1 | 0 | -1 | -2 | -1 | 0 | 1 |
| A: -1 (11) | Re: 0 | 1 | 0 | -1 | -2 | -1 | 0 | 1 | 2 |
| B: +1 (01) | Im: 0 | 1 | 2 | 1 | 0 | -1 | -2 | -1 | 0 |

Fig. 25

Fig. 26

Fig. 27

43

Fig. 28

Fig. 29

Fig.30

Fig.31

Fig. 32

0 011 878